(19) **Europäisches Patentamt / European Patent Office / Office européen des brevets**

(11) **EP 2 746 759 B1**

(12) **EUROPEAN PATENT SPECIFICATION**

(45) Date of publication and mention
of the grant of the patent:
**07.09.2016 Bulletin 2016/36**

(51) Int Cl.:
*C12Q 1/00* (2006.01)    *G01N 27/327* (2006.01)

(21) Application number: **13196640.0**

(22) Date of filing: **11.12.2013**

(54) **Method of detecting concentration of an analyte in a sample with a test strip**

Verfahren zur Messung der Konzentration eines Analyts in einer Probe mittels eines Teststreifens

Procédé de détection d'une concentration d'un analyte d'un échantillon en utilisant d'une bande de test

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.12.2012 US 201261745644 P**
**09.12.2013 TW 102145169**

(43) Date of publication of application:
**25.06.2014 Bulletin 2014/26**

(73) Proprietor: **Tyson Bioresearch, Inc.**
**Miaoli Hsien 35053 (TW)**

(72) Inventors:
• **Lee, Cheng-Che**
**Hsinchu County 302 (TW)**

• **Chen, Wen-Huang**
**New Taipei City 251 (TW)**
• **Tsai, Han-Ching**
**New Taipei City 242 (TW)**
• **Yang, Chen-Yu**
**Miaoli County 351 (TW)**

(74) Representative: **Becker Kurig Straus**
**Patentanwälte**
**Bavariastrasse 7**
**80336 München (DE)**

(56) References cited:
**WO-A1-2012/084194    WO-A1-2013/164632**
**WO-A1-2014/016578    US-A1- 2008 000 780**
**US-A1- 2011 155 588**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Field of the Invention

The present invention relates to a method of a test strip detecting concentration of an analyte of a sample and a test strip with three electrodes according to the claims.

Background of the Invention

Electrochemical biosensors have been commonly used to determine the concentration of various analytes from test samples, such as glucose, uric acid and cholesterol in biological fluids. For example, in biological sample testing, the test strip may be inserted into a glucose meter, and a fluid sample is dropped on a test strip and introduced into a sample chamber to determine the concentration of the analyte in the biological sample.

In the recent years, numbers of people with diabetes are growing. Glucose concentration monitoring is important of everyday life for diabetic patents. Routine tests 3-4 times every day and controlling stabile blood glucose concentration can reduce the risk of serious damages, such as vision loss and kidney failure. The accurate measurement of blood glucose is expected.

However, biosensors may provide the testing results including the multiple analytic errors. When the testing sample is whole blood, these error sources may come from physical characteristics of the whole blood (e.g. interferences), environmental factors (e.g. temperature), and operating conditions (e.g. under-fill). The physical characteristics of the blood include interferences, such as hematocrit (ratio of red blood cell volume to the total blood volume), ascorbic acid, uric acid, cholesterol, and the like.

For example, the normal hematocrit range for a typical human is about 35% to 55%. However, in some special cases, the hematocrit may range from 10% to 70% and induce the large error in the blood glucose measurement. At high hematocrit, the red blood cell may hinder the reaction of enzyme and mediators, and even reduce the diffusion rate of the mediators to the working electrode, resulting in the low blood glucose reading. Conversely, the low hematocrit may result in the high blood glucose reading.

There are many methods to minimize the analytic error of the hematocrit effect. For example, U.S. Pat. No. 5951836 disclosed a reagent formulation using silica particle to filter red blood cell. U.S. Pat. No. 5628890 disclosed reducing the hematocrit effect by using wide spacing in combination with mesh layers to distribute the blood sample. U.S. Pat. No. 8388821 disclosed a method for hematocrit correlated measurement by providing a plurality of microelectrodes on a working electrode. U.S. Pub. No. 2011/0139634 disclosed hematocrit-corrected analyte concentration by using two electrode sets, which apply DC signal and AC signal, separately. The prior methods have some disadvantages, such as high manufacturing cost, a complex process and a large sample amount.

Besides, temperature during the measurement is another analytic error source. Since the enzyme reaction is a temperature-dependent reaction, the temperature change during the measurement has an influence on the measurement accuracy.

Further, focusing on the prior art proposed for detecting concentration of an analyte of a sample, WO 2012/084194 A1 (D1). The second DC signal, or analysis signal, is used to analyze the analyte levels, which can be determined by compensating for reaction time and/or various sources of blank current based on the first response to the first pulse," wherein various sources of blank current include reduced mediator, impurities in the reagent, interfering compounds present in the blood (electroactive), and other interfering sugars, but no hematocrit and temperature. Further, it also discloses "the effects of the confounding factors can be eliminated by measuring the impedance of the blood sample to an AC excitation, either alone or in combination with a DC excitation," wherein (alternating current) AC signals exist between the first DC signal and the second DC signal. Such a technique includes applying an electrical potential signal having an AC component to the sample." That is to say, D1 cannot eliminate the influences from temperature and hematocrit without AC component. Therefore, D1 needs more complicated signals (DC signals and AC signals) to eliminate the influences from reduced mediator, impurities in the reagent, interfering compounds present in the blood, other interfering sugars, temperature and hematocrit.

To sum up, the above mentioned methods provided by the prior art are not good choices for a user.

Summary of the Invention

The present invention aims at providing a method of a test strip with three electrodes detecting concentration of an analyte of a sample that can utilize an electrical signal having different polarities during different periods to detect concentration of an analyte of a sample.

This is achieved by a method of a test strip detecting concentration of an analyte of a sample according to the claims. The dependent claims pertain to corresponding further developments and improvements.

As will be seen more clearly from the detailed description following below, the claimed method of a test strip detecting concentration of an analyte of a sample is disclosed. The method includes placing the sample in a reaction region of the test strip, wherein the analyte reacts with an enzyme to generate a plurality of electrons, and the plurality of electrons are transferred to a working electrode of the reaction region through a mediator; applying an electrical signal to the working electrode; measuring a first current through the working electrode during a first period; the mediator generating during a second period an intermediate according to the electrical signal; measuring during a third period a second current through the working electrode; calculating initial concentration of the analyte according to the first current; calculating a diffusion factor of the intermediate in the sample according to the second current; and correcting the initial concentration to generate new concentration of the analyte according to the diffusion factor.

Brief Description of the Drawings

The present invention is further illustrated by way of example, taking reference to the accompanying drawings thereof:

FIG. 1 is an explosion diagram illustrating a test strip,
FIG. 2 is a diagram illustrating a cross-section of the test strip,
FIG. 3 is a diagram illustrating a test strip with three electrodes,
FIG. 4 is a diagram illustrating a test strip with three electrodes,
FIG. 5 is a diagram illustrating a test strip with three electrodes, a reaction region, and a meter,
FIG. 6 is a diagram illustrating a test strip with three electrodes, a reaction region, and a meter,
FIG. 7 is a diagram illustrating a test strip with three electrodes, a reaction region, and a meter,
FIG. 8 is a diagram illustrating a structure of the test strip,
FIG. 9 is a diagram illustrating a test strip with three electrodes,
FIG. 10 is a diagram illustrating the voltage drop between the working electrode and the reference electrode during a first period, a second period, and a third period,
FIG. 11 is diagram illustrating distribution of a mediator and a reduced state mediator in the reaction region being changed with the voltage drop between the working electrode and the reference electrode when the sample is placed in the reaction region,
FIG. 12 is diagram illustrating distribution of a mediator and a reduced state mediator in the reaction region being changed with the voltage drop between the working electrode and the reference electrode when the sample is placed in the reaction region,
FIG. 13 is diagram illustrating distribution of a mediator and a reduced state mediator in the reaction region being changed with the voltage drop between the working electrode and the reference electrode when the sample is placed in the reaction region,
FIG. 14 is diagram illustrating distribution of a mediator and a reduced state mediator in the reaction region being changed with the voltage drop between the working electrode and the reference electrode when the sample is placed in the reaction region,
FIG. 15 is diagram illustrating relationships between the diffusion factor of the mediator and the current generated by the working electrode under different interfering substances,
FIG. 16 is diagram illustrating relationships between the diffusion factor of the mediator and the current generated by the working electrode under different interfering substances,
FIG. 17 is diagram illustrating relationships between the diffusion factor of the mediator and the current generated by the working electrode under different interfering substances,
FIG. 18 is diagram illustrating a voltage drop between the working electrode and the reference electrode during the first period, the second period, and the third period according to different embodiments of the present invention,
FIG. 19 is diagram illustrating a voltage drop between the working electrode and the reference electrode during the first period, the second period, and the third period according to different embodiments of the present invention,
FIG. 20 is diagram illustrating a voltage drop between the working electrode and the reference electrode during the first period, the second period, and the third period according to different embodiments of the present invention,
FIG. 21 is diagram illustrating a voltage drop between the working electrode and the reference electrode during the first period, the second period, and the third period according to different embodiments of the present invention,
FIG. 22 is diagram illustrating a voltage drop between the working electrode and the reference electrode during the first period, the second period, and the third period according to different embodiments of the present invention,
FIG. 23 is diagram illustrating a voltage drop between the working electrode and the reference electrode during the first period, the second period, and the third period according to different embodiments of the present invention,
FIG. 24 is diagram illustrating a voltage drop between the working electrode and the reference electrode during the first period, the second period, and the third period according to different embodiments of the present invention,

FIG. 25 is a diagram illustrating currents generated by the working electrode corresponding to different samples under the voltage drop between the working electrode and the reference electrode in FIG. 10,

FIG. 26 is a diagram illustrating relationships between biases and concentration of analytes with different hematocrits not corrected by the present invention,

FIG. 27 is a diagram illustrating relationships between biases and concentration of analytes with different hematocrits corrected by the present invention,

FIG. 28 is a flowchart illustrating a method of a test strip detecting concentration of an analyte of a sample according to another embodiment of the present invention, and

FIG. 29 is a flowchart illustrating a method of utilizing a test strip to detect diffusion factor of a mediator in a sample according to another embodiment of the present invention.

Detailed Description

The present invention will now be described through the following embodiments. It is understood by those skilled in the art that the embodiments described below are only for illustration purpose and no limitation of scope of the invention is intended.

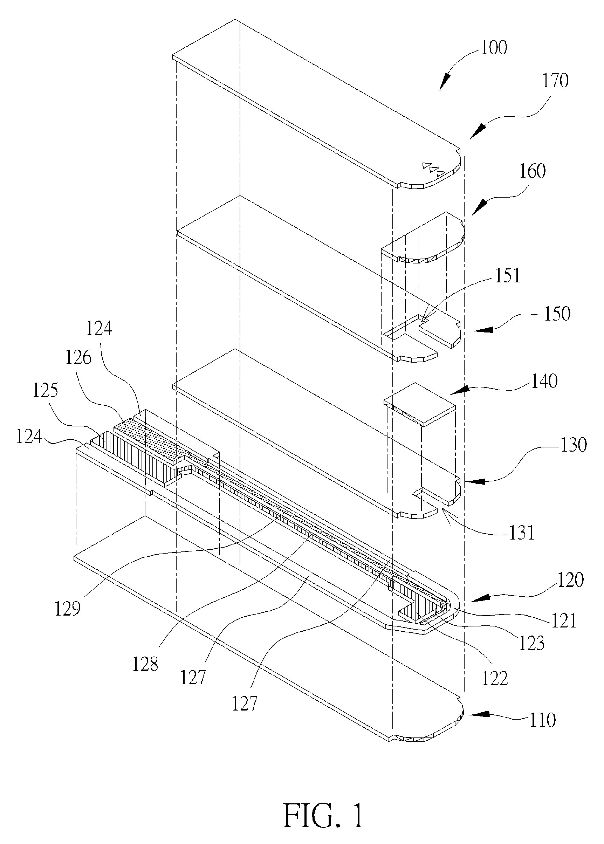

FIG. 1 is an explosion diagram illustrating a test strip 100 to be used in the method. As shown in FIG. 1, the test strip 100 may include a substrate 110, an electrode layer 120, an insulating layer 130, a reagent 140, a spacer 150, a first cover layer 160, and a second cover layer 170, wherein the substrate 110 can be made of a plastic material (e.g. polyethylene terephthalates (PET), vinyl polymers, polyimides, or polyesters).

As shown in FIG. 1, the substrate 110 carries the electrode layer 120, wherein electrode layer 120 has a working electrode 121, a counter electrode 122, and a reference electrode 123, and the working electrode 121, the counter electrode 122, and the reference electrode 123 are formed on a first end of the electrode layer 120. The working electrode 121, the counter electrode 122, and the reference electrode 123 can be formed by laser scribing on the conductive electrode layer 120 or by screen printing onto the substrate 110. A second end of the electrode layer 120 can provide a plurality of pads 124, 125, 126, wherein the plurality of pads 124, 125, 126 are used for electrical coupling to a meter. An electrode track 127 can provide an electrically continuous pathway from the working electrode 121 to the pad 124. Similarly, an electrode track 128 provides an electrically continuous pathway from the counter electrode 122 to the pad 125, and an electrode track 129 provides an electrically continuous pathway from the reference electrode 123 to the pad 126. The electrode layer 120 can be made of conductive materials provided by the prior art (e.g. gold, platinum, sliver, carbon and carbon/silver).

An insulating layer 130 can be used for protecting the electrode tracks 127, 128, 129 and defining an effective area of a reaction region. A notch 131 may be located on a front section of the insulating layer 130 for exposing portions of the working electrode 121, the counter electrode 122, and the reference electrode 123, wherein the exposed portions of the working electrode 121, the counter electrode 122, and the reference electrode 123 and the reagent 140 can be combined to form the reaction region. The insulating layer 130 can be made of ink, ultraviolet radiation (UV) polymer or the like, and can be formed on the electrode layer 120 through the screen printing.

The reagent 140 may be located on the exposed portions of the working electrode 121, the counter electrode 122, and the reference electrode 123 exposed by the notch 131. The choice of the reagent 140 depends on the specific analyte and is well known by those skilled in the art. In one embodiment of the present invention, the reagent 140 is used for measuring glucose from a human blood sample. A non-limiting reagent can include an enzyme, electron mediators, stabilizers and binders, wherein the enzyme can be glucose oxidase or glucose dehydrogenase. The electron mediator is an electron acceptor and capable of transferring electrons between the enzyme and the working electrode 121. Typically, the mediator can be ferrocene, potassium ferricyanide or other ferrocene derivatives. In one embodiment of the present invention, the reaction region at the reagent 140 is where the glucose in the human blood sample reacts with the enzyme, electrons are transferred through the mediators to the working electrode 121, and an electrical response is generated.

The spacer 150 overlies the substrate 110 and can define height of the sample-receiving chamber. In one embodiment of the present invention, the spacer 150 has a T-shaped channel 151 located at a front section of the spacer 150.

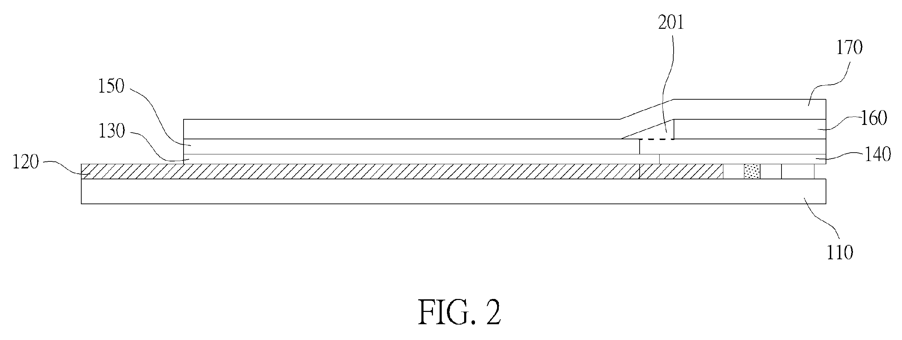

The first cover layer 160 is attached on a part of the spacer 150 for forming a top surface of the sample-receiving chamber. A bottom of the first cover layer 160 includes a hydrophilic coating (not shown in FIG. 1). When the human blood sample enters the sample-receiving chamber, the hydrophilic coating can help a capillary action and increase a speed of movement of the human blood sample. As shown in FIG. 1, a final layer of the test strip 100 is the second cover layer 170. The second cover layer 170 includes a transparent window that allows a user to visually confirm if the human blood sample enters the sample-receiving chamber. As shown in FIG. 2, the first cover layer 160, the second cover layer 170, and the spacer 150 form a opening 201, wherein when the human blood sample enters the sample-receiving chamber, the opening 201 allows air to escape from the interior of the sample-receiving chamber.

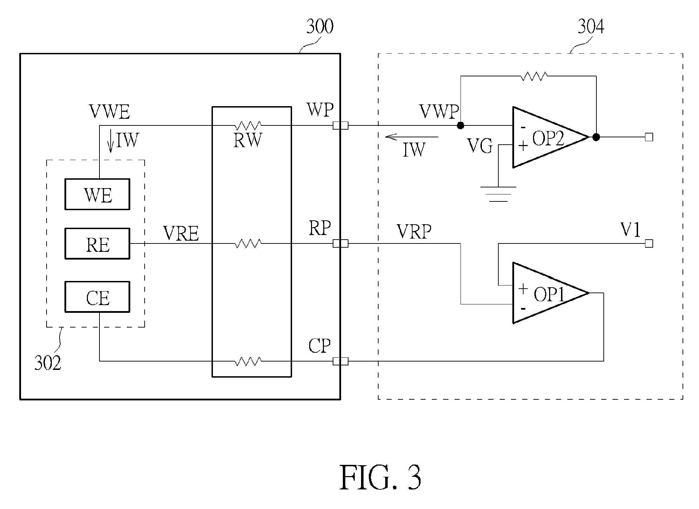

In general biochemical measurements, a meter applies an electrical signal to a working electrode of a test strip, and then the meter measures a current generated by the working electrode for the following measurements. FIG. 3 is a diagram illustrating a test strip 300 with three electrodes, a reaction region 302, and a meter 304, wherein the meter 304 is electrically connected to a working electrode WE, a reference electrode RE, and a counter electrode CE of the reaction region 302 through pads WP, RP, CP, respectively, and OP1, OP2 are operational amplifiers. As shown in FIG. 3, when a sample is placed in the reaction region 302 of the test strip 300 coupled to the meter 304, the meter 304 can apply a fixed voltage (e.g. ground potential VG) to the operational amplifier OP2 to measure a current IW generated by the working electrode WE. Because an electrode track between the working electrode WE and the pad WP has an equivalent resistor RW, a real voltage VWE of the working electrode WE can be determined by equation (1):

$$
\begin{aligned}
VWP &= VG \\
VWE &= VWP - IW \times RW \qquad (1) \\
&= VG - IW \times RW
\end{aligned}
$$

Furthermore, a voltage drop VWR (equal to the electrical signal applied to the working electrode WE) between the working electrode WE and the reference electrode RE can be determined by equation (2):

$$
\begin{aligned}
VWR &= VWE - VRE \\
&= VG - IW \times RW - VRE \\
&= VG - VRP - IW \times RW \qquad (2) \\
&= VG - V1 - IW \times RW
\end{aligned}
$$

As shown in equation (2), VRE is a voltage of the reference electrode RE, VRP is a voltage of the pad RP, and V1 is a reference voltage inputted to the operational amplifier OP1. As shown in equation (2), the voltage drop VWR between the working electrode WE and the reference electrode RE is influenced by the current IW. Because the voltage drop VWR between the working electrode WE and the reference electrode RE is influenced by the current IW, the meter 304 can provide a stable voltage to the reference electrode RE, but cannot provide a stable voltage to the working electrode WE for the accurate subsequent measurements. Therefore, the test strip with three electrodes (e.g. the test strip 300 as shown in FIG. 3) provided by the prior art cannot meet requirements of a user.

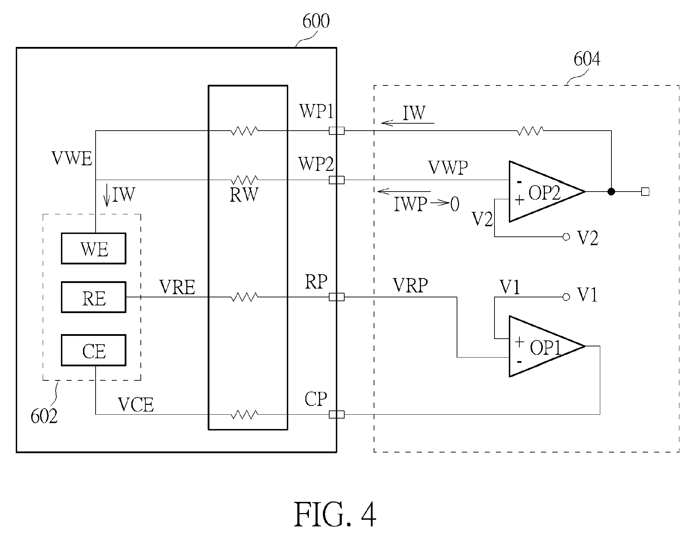

FIG. 4 is a diagram illustrating a test strip 600 with three electrodes, a reaction region 602, and a meter 604 to be used in the method wherein the meter 604 is electrically connected to a working electrode WE of the reaction region 602 through pads WP1, WP2, a reference electrode RE of the reaction region 602 through a pad RP, and a counter electrode CE of the reaction region 602 through a pad CP. Furthermore, OP1, OP2 in the meter 604 are operational amplifiers, and the reaction region 602 is coated with an enzyme. As shown in FIG. 4, when a sample is placed in the reaction region 602 of the test strip 600 coupled to the meter 604, the meter 604 can apply a voltage V2 to the operational amplifier OP2 to measure a current IW generated by the working electrode WE, wherein the sample covers at least the working electrode WE. Because an electrode track between the working electrode WE and the pad WP2 has an equivalent resistor RW, a real voltage VWE of the working electrode WE can be determined by equation (3):

$$
\begin{aligned}
VWE &= VWP - IWP \times RW \\
&= VWP \, (\because IWP = 0) \qquad (3) \\
&= V2
\end{aligned}
$$

As shown in equation (3), VWP is a voltage of the pad WP2, and the IWP is a current following through the pad WP2. Furthermore, in FIG. 4, a voltage drop VWR between the working electrode WE and the reference electrode RE (equal to an electrical signal applied to the working electrode WE) can be determined by equation (4):

$$
\begin{aligned}
VWR &= VWE - VRE \\
&= VWP - VRP \qquad (4) \\
&= V2 - V1
\end{aligned}
$$

As shown in equation (4), VRE is a voltage of the reference electrode RE, VRP is a voltage of the pad RP, and V1 is a reference voltage inputted to the operational amplifier OP1. As shown in equation (4), the voltage drop VWR between the working electrode WE and the reference electrode RE (equal to the electrical signal applied to the working electrode WE) is not influenced by the current IW. Because the voltage drop VWR between the working electrode WE and the reference electrode RE is not influenced by the current IW, the meter 604 not only can provide a stable voltage to the reference electrode RE, but can also provide a stable voltage to the working electrode WE for the accurate subsequent measurements. Therefore, the test strip with 4 pads (e.g. the test strip 600 as shown in FIG. 4) provided by the present invention can meet the requirements of the user.

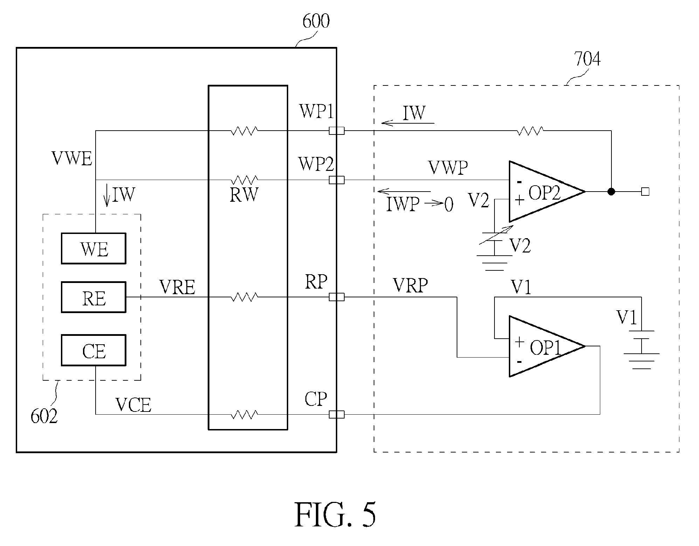

FIG. 5 is a diagram illustrating a test strip 600 with three electrodes, a reaction region 602, and a meter 704 to be used in the method, wherein the meter 704 is electrically connected to a working electrode WE of the reaction region 602 through pads WP1, WP2, a reference electrode RE of the reaction region 602 through a pad RP, and a counter electrode CE of the reaction region 602 through a pad CP. Furthermore, OP1, OP2 in the meter 704 are operational amplifiers. As shown in FIG. 5, a difference between the meter 704 and the meter 604 in FIG. 4 is that a voltage V2 applied to the operational amplifier OP2 by the meter 704 is a variable voltage and a voltage V1 applied to the operational amplifier OP1 by the meter 704 is a fixed voltage. Furthermore, subsequent operational principles of the test strip 600, the reaction region 602, and the meter 704 in FIG. 5 are the same as those of the test strip 600, the reaction region 602, and the meter 604 in FIG. 4, so further description thereof is omitted for simplicity.

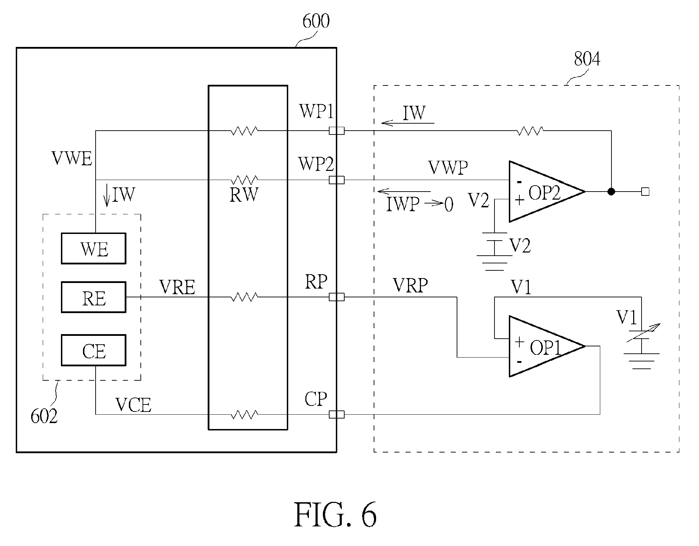

FIG. 6 is a diagram illustrating a test strip 600 with three electrodes, a reaction region 602, and a meter 804 to be used in the method, wherein the meter 804 is electrically connected to a working electrode WE of the reaction region 602 through pads WP1, WP2, a reference electrode RE of the reaction region 602 through a pad RP, and a counter electrode CE of the reaction region 602 through a pad CP. Furthermore, OP1, OP2 in the meter 804 are operational amplifiers. As shown in FIG. 6, a difference between the meter 804 and the meter 604 in FIG. 4 is that a voltage V2 applied to the operational amplifier OP2 by the meter 804 is a fixed voltage and a voltage V1 applied to the operational amplifier OP1 by the meter 804 is a variable voltage. Furthermore, subsequent operational principles of the test strip 600, the reaction region 602, and the meter 804 in FIG. 6 are the same as those of the test strip 600, the reaction region 602, and the meter 604 in FIG. 4, so further description thereof is omitted for simplicity.

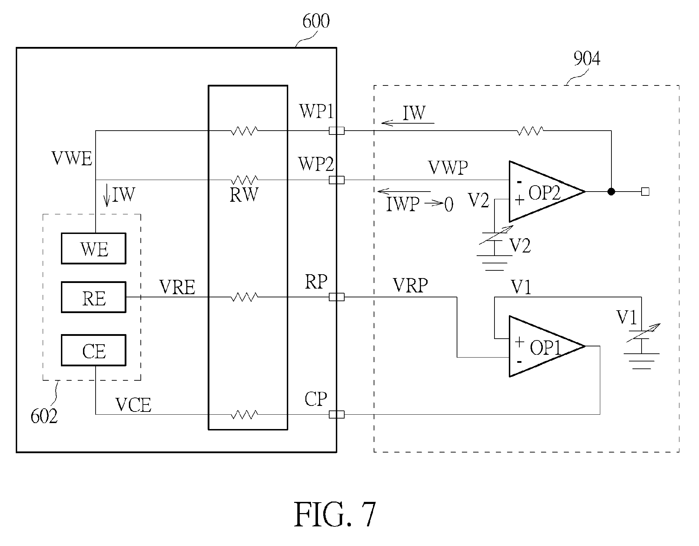

FIG. 7 is a diagram illustrating a test strip 600 with three electrodes, a reaction region 602, and a meter to be used in the method, wherein the meter 904 is electrically connected to a working electrode WE of the reaction region 602 through pads WP1, WP2, a reference electrode RE of the reaction region 602 through a pad RP, and a counter electrode CE of the reaction region 602 through a pad CP. Furthermore, OP1, OP2 in the meter 904 are operational amplifiers. As shown in FIG. 7, a difference between the meter 904 and the meter 604 in FIG. 4 is that a voltage V2 applied to the operational amplifier OP2 by the meter 904 is a variable voltage and a voltage V1 applied to the operational amplifier OP1 by the meter 904 is a variable voltage. Furthermore, subsequent operational principles of the test strip 600, the reaction region 602, and the meter 904 in FIG. 7 are the same as those of the test strip 600, the reaction region 602, and the meter 604 in FIG. 4, so further description thereof is omitted for simplicity.

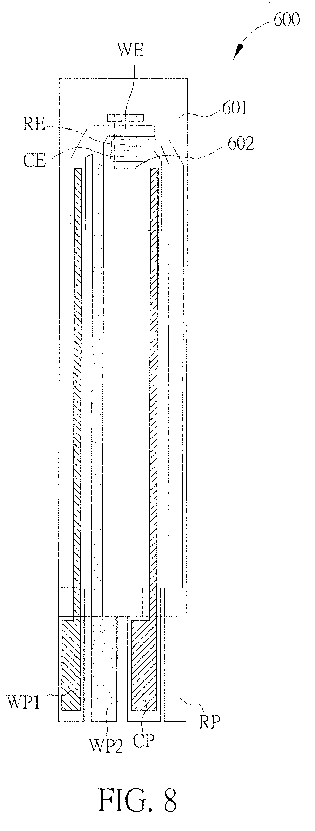

FIG. 8 is a diagram illustrating a structure of the test strip 600. As shown in FIG. 8, the test strip 600 includes a substrate 601, the reaction region 602, the working electrode WE, the reference electrode RE, and the counter electrode CE, wherein the working electrode WE connects to the pads WP1, WP2, the reference electrode RE connects to the pad RP, and the counter electrode CE connects to the pad CP. Furthermore, the substrate 601 is made of an insulting material (e.g. polyethylene terephthalate (PET) or insulting materials as like). As shown in FIG. 8, the working electrode WE, the reference electrode RE, and the counter electrode CE are formed on the substrate 601 and the reaction region 602 is formed on a first end of the substrate 601, wherein the working electrode WE, the reference electrode RE, and the counter electrode CE are made of conductive materials, wherein the conductive materials include gold, platinum, silver or graphite. The present invention is not limited to the conductive materials including gold, platinum, silver or graphite. Furthermore, the pads WP1, WP2, RP, CP are formed on a second end of the substrate 601, wherein the second end of the substrate 601 is opposite to the first end of the substrate 601. As shown in FIG. 8, the pads WP1, WP2 are formed on a left side of the substrate 601 (wherein positions of the pads WP1, WP2 can be exchanged with each other), the pads RP, CP are formed on a right side of the substrate 601, and the pad CP is located between the pad WP2 and the pad RP. Furthermore, in another embodiment of the present invention, the pads WP1, WP2 are formed on the right side of the substrate 601, the pads RP, CP are formed on the left side of the substrate 601, and the pad CP is located between the pad WP2 and the pad RP. Furthermore, in the reaction region 602, the reference electrode RE is located between the counter electrode CE and the working electrode WE.

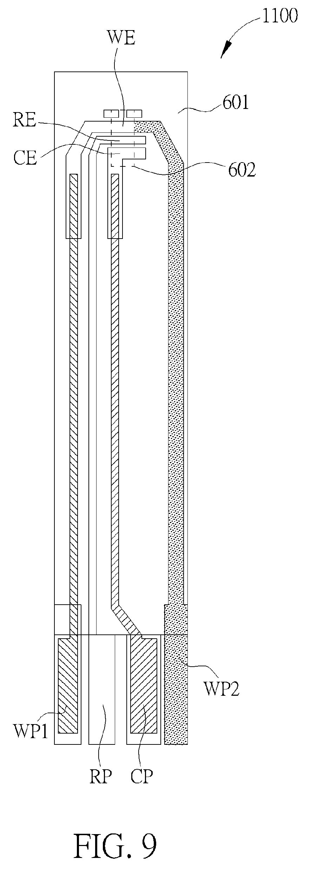

Furthermore, FIG. 9 is a diagram illustrating a test strip 1100 with three electrodes to be used in the method. As shown in FIG. 9, a difference between the test strip 1100 and the test strip 600 in FIG. 8 is that a pad WP1 and a pad WP2 coupled to a working electrode WE are formed on a left side and a right side of a substrate 601 respectively, a pad RP coupled to a reference electrode RE and a pad CP coupled to a counter electrode CE are formed on a middle of the substrate 601, and the pad RP is located between the pad WP1 and the pad CP. Furthermore, subsequent operational principles of the test strip 1100 are the same as those of the test strip 600, so further description thereof is omitted for simplicity.

EP 2 746 759 B1

Furthermore, as shown in FIG. 4, when a sample (e.g. blood) is placed in the reaction region 602 of the test strip 600 coupled to the meter 604 through the pads WP1, WP2, RP, CP, the meter 604 applies the voltage V2 to the operational amplifier OP2 and the reference voltage V1 to the operational amplifier OP1 to measure the current IW of the working electrode WE. As shown in equation (4), when the sample is placed in the reaction region 602 of the test strip 600, because the voltage drop VWR between the working electrode WE and the reference electrode RE (equal to the electrical signal applied to the working electrode WE) is not influenced by the current IW, the meter 604 can provide the stable voltage drop VWR and accurately measure the current IW generated by the working electrode WE through the pad WP1 for subsequent calculation. Furthermore, subsequent operational principles of the meter 704, the meter 804, and the meter 904 are the same as those of the meter 604, so further description thereof is omitted for simplicity.

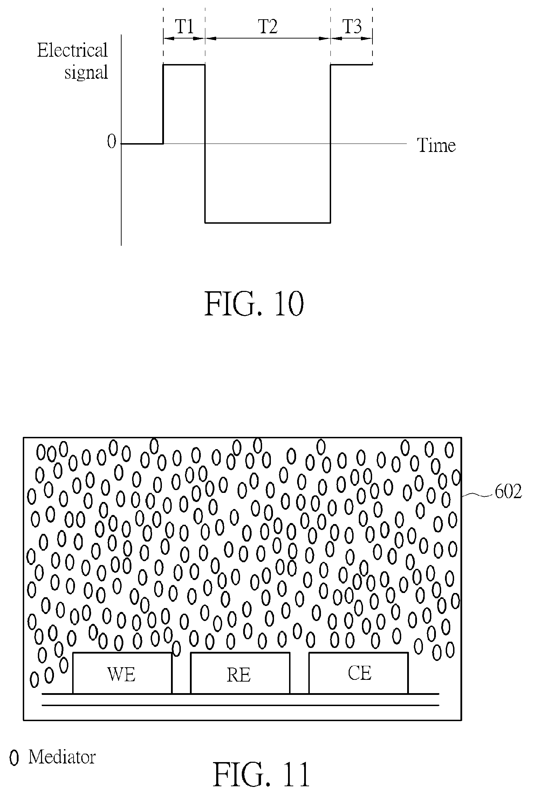

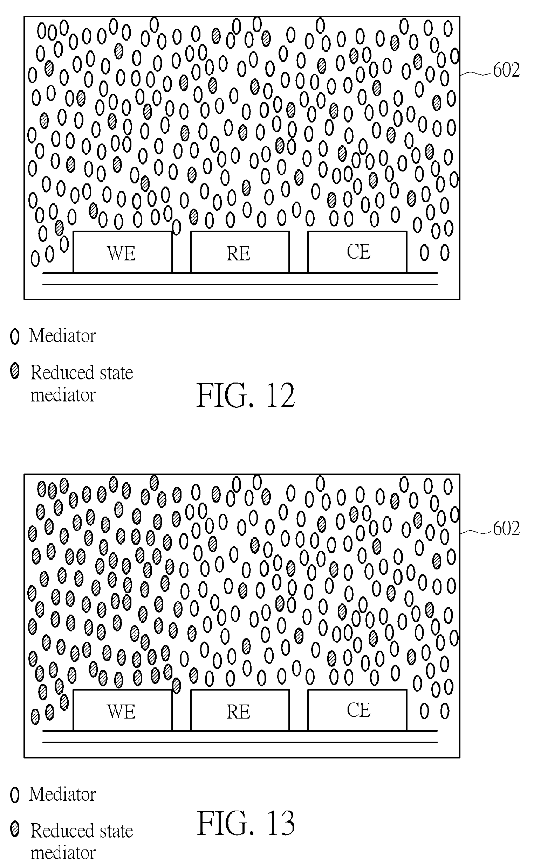

FIG. 10 is a diagram illustrating the voltage drop VWR between the working electrode WE and the reference electrode RE (equal to the electrical signal applied to the working electrode WE) during a first period T1, a second period T2, and a third period T3, and FIG. 11 to FIG. 14 are diagrams illustrating distribution of a mediator and a reduced state mediator in the reaction region 602 being changed with the voltage drop VWR between the working electrode WE and the reference electrode RE when the sample (e.g. blood) is placed in the reaction region 602, wherein the mediator can be pre-coated on the surface of the reaction region 602 or be added to the reaction region 602 when the sample is placed in the reaction region 602. As shown in FIG. 10 and FIG. 11, when the sample (including an analyte (e.g. blood sugar)) is placed in the reaction region 602, the mediator in the sample can directly or indirectly seize electrons from the analyte to become a reduced state mediator, wherein concentration of the mediator is much greater than concentration of the analyte. Therefore, as shown in FIG. 10 and FIG. 12, because the electrical signal applied to the working electrode WE is a positive polarity signal during the first period T1, the reduced state mediator can transfer electrons to the working electrode WE through a diffusion effect. That is to say, the working electrode WE can generate the current IW (first current) through the reduced state mediator during the first period T1, wherein the current IW (first current) during the first period T1 can be used for calculating initial concentration of the analyte. For example, if the sample is blood and the analyte in the sample is blood sugar, the mediator in the reaction region 602 can be potassium ferricyanide, wherein the potassium ferricyanide can directly or indirectly react with the blood sugar through the enzyme to generate reduced state potassium ferrocyanide. During the first period T1, the positive polarity signal applied to the working electrode WE can make the potassium ferrocyanide diffuse to the working electrode WE to generate the current IW (first current). The present invention is not limited to the mediator in the sample directly or indirectly seizing electrons from the analyte to be a reduced state mediator. That is to say, the mediator in the sample can also directly or indirectly transfer electrons to the analyte to become an oxidized state mediator.

As shown in FIG. 10 and FIG. 13, because the concentration of the mediator is much greater than the concentration of the analyte, the majority of mediator not reacted with the analyte can generate reduction reaction on a surface of the working electrode WE when the electrical signal applied to the working electrode WE is a negative polarity signal during the second period T2, resulting in the high concentration reduced state mediator (that is, the intermediate) being accumulated on the surface of the working electrode WE, wherein the concentration of the reduced state mediator accumulated on the surface of the working electrode WE is not influenced by the concentration of the analyte (e.g. blood sugar). For example, if the mediator is potassium ferricyanide, the negative polarity signal applied to the working electrode WE during the second period T2 can make potassium ferricyanide not reacted with the analyte (e.g. blood sugar) reduce to potassium ferrocyanide, wherein potassium ferrocyanide is the intermediate of the present invention, and concentration of potassium ferrocyanide is not influenced by the analyte (e.g. blood sugar). In another embodiment of the present invention, because the electrical signal applied to the working electrode WE is a positive polarity signal during the second period T2, the majority of mediator not reacted with the analyte can generate oxidation reaction on the surface of the working electrode WE. That is to say, the high concentration oxidized state mediator can be accumulated on the surface of the working electrode WE. Furthermore, the present invention is not limited to the electrical signal applied to the working electrode WE being a voltage signal during the second period T2, that is, the electrical signal applied to the working electrode WE can also be a current signal during the second period T2.

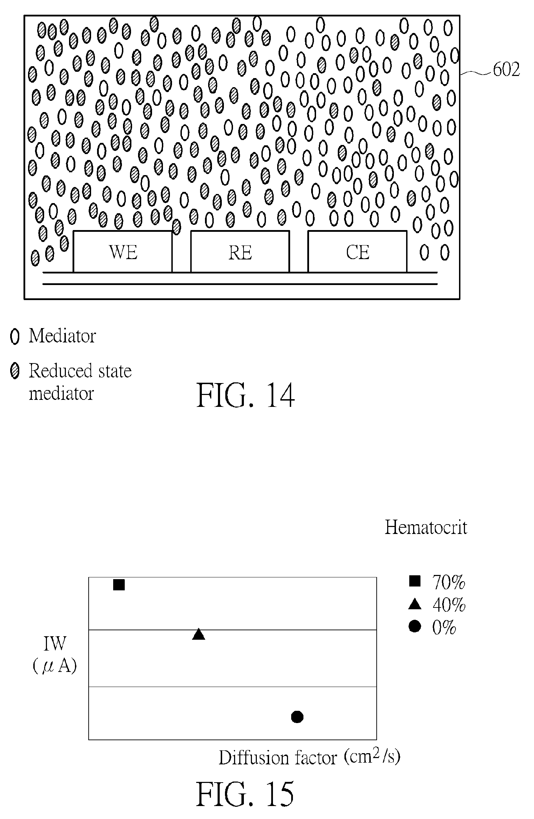

Diffusion behavior of the mediator in the sample corresponds to a diffusion factor of the sample, wherein the diffusion factor is a function corresponding to a combination of temperature, viscosity, hematocrit, lipemic and ionic strength of the sample. The present invention is not limited to the diffusion factor being a function corresponding to a combination of temperature, viscosity, hematocrit, lipemic and ionic strength of the sample. When the diffusion factor of the mediator in the sample is lower, the reduced state mediator (the intermediate) generated during the second period T2 cannot diffuse easily (as shown in FIG. 14); on the other hand, when the diffusion factor of the mediator in the sample is higher, the reduced state mediator (the intermediate) generated during the second period T2 can diffuse easily. Therefore, during the third period T3 in FIG. 10, when the electrical signal applied to the working electrode WE is a positive polarity signal, the working electrode WE can generate a greater current IW (second current) when the diffusion factor of the mediator in the sample is lower (because the more reduced state mediator can be accumulated on the surface of the working electrode WE, the working electrode WE can receive more electrons, resulting in the working electrode WE generating the greater current IW (second current)), and the working electrode WE can generate a smaller current IW (second current) when the diffusion factor of the mediator in the sample is higher (because the less reduced state mediator can be accumulated on the surface of the working electrode WE, the working electrode WE can receive fewer electrons, resulting in the working electrode WE generating the smaller current IW (second current)). Furthermore, in another embodiment of the present invention, the electrical signal applied to the working electrode WE during the third period T3 is a negative polarity signal opposite to a positive polarity signal during the second period T2.

Furthermore, during the first period T1, the second period T2, and the third period T3, the counter electrode CE is used for receiving a floating voltage VCE provided by the operational amplifier OP1 to satisfy the current IW generated by the working electrode WE. Therefore, a reaction material can be coated on a surface of the counter electrode CE (or the counter electrode CE can directly react with the electrical signal applied to the working electrode WE) to prevent a voltage of the counter electrode CE from being increased too high during the first period T1, the second period T2, and the third period T3, wherein an oxidized-reduced state of the reaction material coated on the surface of the counter electrode CE is opposite to an original oxidized-reduced state of the mediator in the sample.

Furthermore, as shown in FIG. 4 to FIG. 7, the reference electrode RE is coupled to a negative input terminal of the operational amplifier OP1 (that is, no current flowing through the reference electrode RE) and the reference electrode RE is between the working electrode WE and the counter electrode CE, so the reference electrode RE can prevent the reaction material coated on the surface of the counter electrode CE or a product generated by the counter electrode CE from being diffused to the working electrode WE. That is to say, the reference electrode RE can prevent the counter electrode CE from influencing the current IW generated by the working electrode WE during the first period T1, the second period T2, and the third period T3.

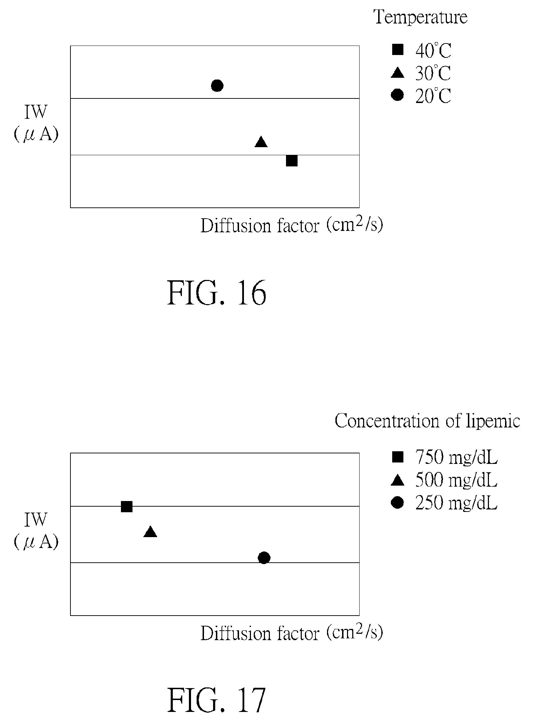

FIG. 15 to FIG. 17 are diagrams illustrating relationships between the diffusion factor of the mediator and the current IW (second current) generated by the working electrode WE under different interfering substances. As shown in FIG. 15, when hematocrit HCT of the sample is higher (the mediator has the lower diffusion factor), the current IW generated by the working electrode WE is greater. For example, the current IW generated by the working electrode WE when the hematocrit HCT of the sample is 70% is greater than the current IW generated by the working electrode WE when the hematocrit HCT of the sample is 40%. As shown in FIG. 16, when temperature of the sample is lower (the mediator has the lower diffusion factor), the current IW generated by the working electrode WE is greater. For example, the current IW generated by the working electrode WE when the temperature of the sample is 20°C is greater than the current IW generated by the working electrode WE when the temperature of the sample is 30°C. As shown in FIG. 17, when concentration of lipemic (triglyceride) of the sample is higher (the mediator has the lower diffusion factor), the current IW generated by the working electrode WE is greater. For example, the current IW generated by the working electrode WE when the concentration of the lipemic of the sample is 750mg/dL is greater than the current IW generated by the working electrode WE when the concentration of the lipemic of the sample is 500mg/dL. Thus, during the third period T3 in FIG. 10, the meter 604 can calculate the diffusion factor of the intermediate (the reduced state mediator) in the sample through the current IW generated by the working electrode WE (second current) according to the above mentioned principles, wherein the second current is a diffusion current generated by the intermediate during the third period T3.

Because the concentration of the reduced state mediator on the surface of the working electrode WE is not influenced by the concentration of the analyte (e.g. blood sugar), after the diffusion factor of the mediator in the sample is determined, the meter 604 can correct an error of the concentration of the analyte in the sample to generate new concentration of the analyte according to the diffusion factor of the mediator in the sample, wherein factors causing the error of the concentration of the analyte correspond to a combination of temperature, viscosity, hematocrit, lipemic and ionic strength of the sample.

Furthermore, when the electrical signal applied to the working electrode WE is a voltage, a range of the electrical signal during the first period T1 and a range of the electrical signal during the third period T3 are about between 50mV-1000mV, the best about between 200mV-500mV A range of the electrical signal during the second period T2 is about between -50mV and -1000mV, the best about between -100mV and -500 mV. Further, a range of the second period T2 is about between 0.5 seconds and 10 seconds, the best about between 1 second and 8 seconds. Furthermore, in another embodiment of the present invention, the electrical signal during the second period T2 is a predetermined current.

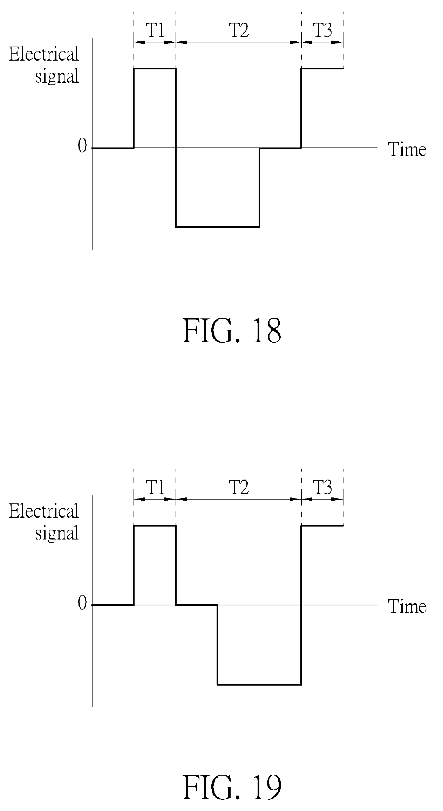

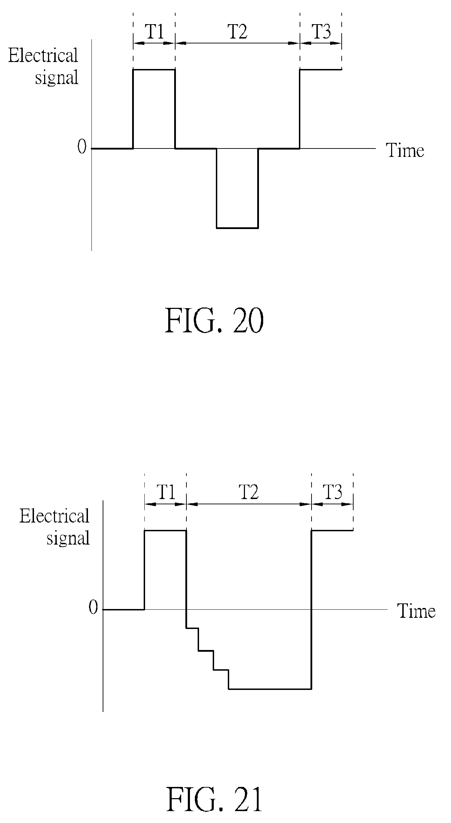

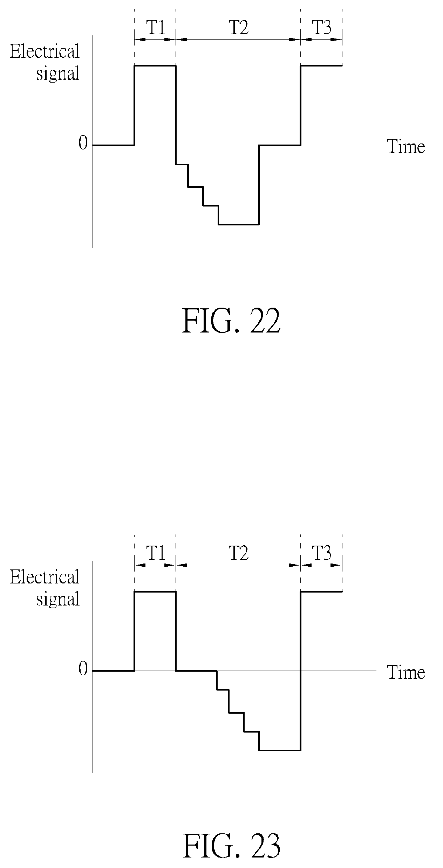

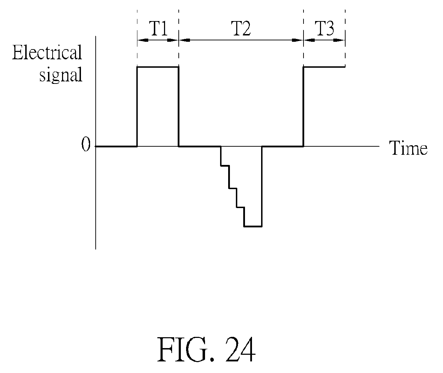

FIG. 18 to FIG. 24 are diagrams illustrating a voltage drop VWR between the working electrode WE and the reference electrode RE (equal to an electrical signal applied to the working electrode WE) during the first period T1, the second period T2, and the third period T3 according to different embodiments, wherein subsequent operational principles of the voltage drop VWR between the working electrode WE and the reference electrode RE during the first period T1, the second period T2, and the third period T3 in FIG. 18 to FIG. 24 are the same as those of the voltage drop VWR between the working electrode WE and the reference electrode RE during the first period T1, the second period T2, and the third period T3 in FIG. 10. Furthermore, as shown in FIG. 18 to FIG. 24, the voltage drop VWR between the working electrode WE and the reference electrode RE can be 0 (non-polarity) and a negative polarity signal during the second period T2, that is, the electrical signal applied to the working electrode WE is 0 and the negative polarity signal.

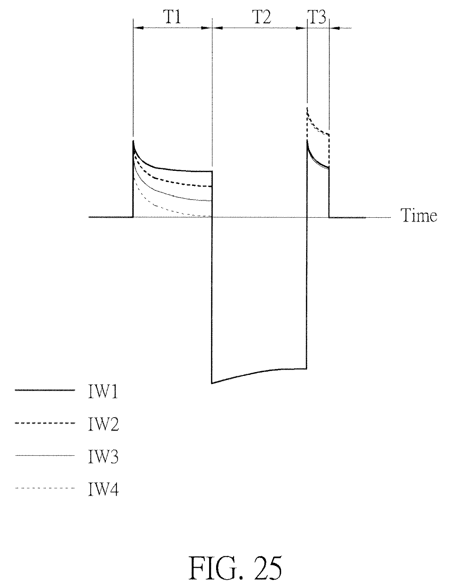

FIG. 25 is a diagram illustrating currents IW1, IW2, IW3, IW4 generated by the working electrode WE corresponding to different samples under the voltage drop VWR between the working electrode WE and the reference electrode RE in FIG. 10. Because principles of other factors (e.g. temperature, viscosity, hematocrit, lipemic and ionic strength of the sample) causing the error of the concentration of the analyte are the same as those of hematocrit, so further description thereof is omitted for simplicity. As shown in FIG. 11 and FIG. 12, when the sample (including blood sugar) is placed in the reaction region 602, the mediator in the sample can directly or indirectly seize electrons from the analyte to become the reduced state mediator, so the working electrode WE can generate the current IW (first current) through the reduced state mediator, wherein the current IW (first current) during the first period T1 can be used for calculating the initial concentration of the analyte. Furthermore, before the high concentration reduced state mediator is not yet generated and accumulated on the surface of the working electrode WE, the current IW (first current) generated by the working electrode WE is greater when the hematocrit of the sample is lower. Therefore, as shown in FIG. 25, during the first period T1, the current IW1 (first current) corresponding to a sample 1 is higher than the current IW2 (first current) corresponding to a sample 2, higher than the current IW3 (first current) corresponding to a sample 3, and higher than the current IW4 (first current) corresponding to a sample 4, wherein the sample 1 has blood sugar concentration (200mg/dL) and hematocrit HCT (10%), the sample 2 has blood sugar concentration (200mg/dL) and the hematocrit HCT (70%), the sample 3 has blood sugar concentration (100mg/dL) and the hematocrit HCT (10%), and the sample 4 has blood sugar concentration (100mg/dL) and the hematocrit HCT (70%). As shown in FIG. 13, during the second period T2, because the electrical signal applied to the working electrode WE is the negative polarity signal, the majority of mediator not reacted with the analyte can generate reduction reaction on the surface of the working electrode WE. That is to say, the high concentration reduced state mediator (the intermediate) is accumulated on the surface of the working electrode WE. The concentration of the reduced state mediator accumulated on the surface of the working electrode WE is not influenced by the concentration of the analyte (e.g. blood sugar), so the current IW (second current) generated by the working electrode WE is lower when the diffusion factor of the mediator in the sample is higher, and the current IW (second current) generated by the working electrode WE is higher when the diffusion factor of the mediator in the sample is lower. Therefore, during the third period T3 in FIG. 25, a difference between the current IW1 (second current) corresponding to the sample 1 and the current IW3 (second current) corresponding to the sample 3 is minor (because the sample 1 and the sample 3 have the same hematocrit HCT (10%)) and a difference between the current IW2 (second current) corresponding to the sample 2 and the current IW4 (second current) corresponding to the sample 4 is minor (because the sample 2 and the sample 4 have the same hematocrit HCT (70%)), and the current IW1 (second current) corresponding to the sample 1 and the current IW3 (second current) corresponding to the sample 3 are less than the current IW2 (second current) corresponding to the sample 2 and the current IW4 (second current) corresponding to the sample 4. Thus, during the third period T3 in FIG. 25, the meter 604 can calculate diffusion factors of the mediator in the samples 1, 2, 3, 4 respectively through the currents IW1, IW2, IW3, IW4 (second currents) generated by the working electrode according to the above mentioned principles. After the diffusion factors of the mediator in the samples 1, 2, 3, 4 are determined, the meter 604 can correct errors of concentration of analytes in the samples 1, 2, 3, 4 to generate new concentration of the analytes in the samples 1, 2, 3, 4 respectively according to the diffusion factors of the mediator in the samples 1, 2, 3, 4.

Furthermore, the present invention is not limited to the oxidized-reduced state of the mediator in FIG. 11 to FIG. 14. That is to say, in another embodiment of the present invention, a new mediator has an oxidized-reduced state opposite to the oxidized-reduced state of the mediator in FIG. 11 to FIG. 14, and a new electrical signal applied to the working electrode WE is opposite to the electrical signal applied to the working electrode WE in FIG. 10.

Furthermore, the test strips 100, 600, 1100 and the meter 604 provided by the present invention can also be integrated into a biometric system, wherein subsequent operational principles of the biometric system can be referred to those of the test strips 100, 600, 1100, and the meter 604, so further description thereof is omitted for simplicity.

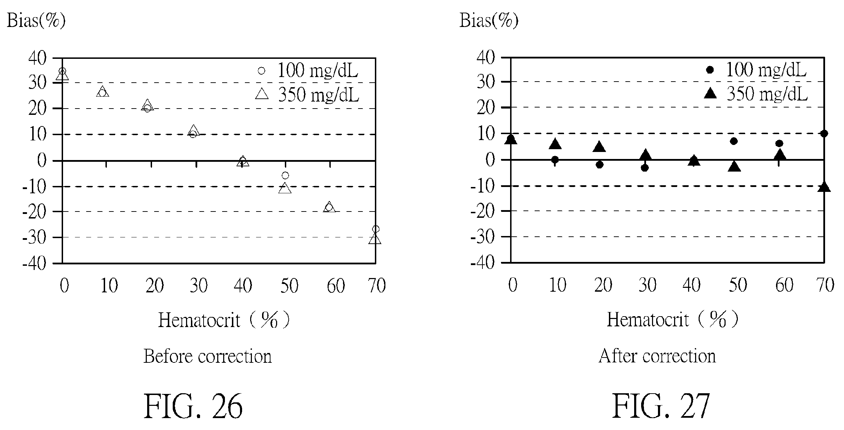

FIG. 26 is a diagram illustrating relationships between biases and concentration of analytes with different hematocrits not corrected by the present invention. As shown in FIG. 26, an analyte with 41% hematocrit is acted as a standard, wherein the biases are differences between each concentration of analyte and the standard, and the concentrations of the analytes (e.g. blood sugar) in samples are 100mg/dL and 350mg/dL. As shown in FIG. 26, when hematocrit in the samples deviates from the standard, greater biases exist. FIG. 27 is a diagram illustrating relationships between biases and concentration of analytes with different hematocrits corrected by the present invention. As shown in FIG. 27, the above mentioned test strip and method provided by the present invention can significantly decrease biases within the whole range (0-70%) of hematocrit to control the biases away from the standard +10% to -10%.

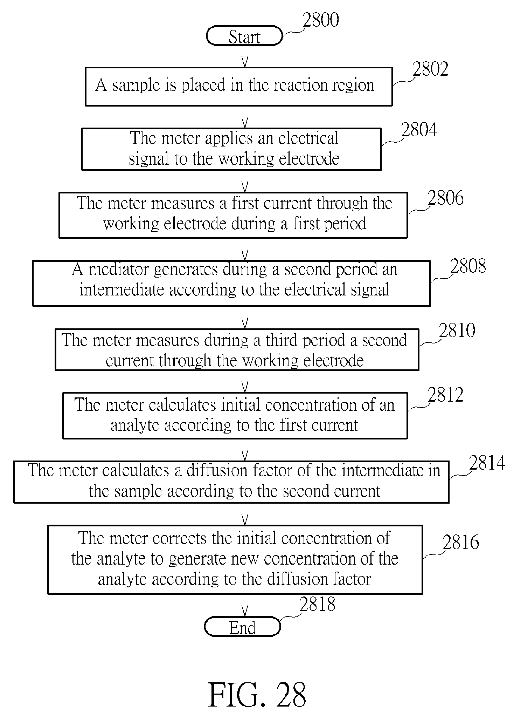

FIG. 28 is a flowchart illustrating a method of a test strip detecting concentration of an analyte of a sample according to another embodiment. The method in FIG. 28 is illustrated using the test strip 600 in FIG. 4. Detailed steps are as follows:

Step 2800:    Start.
Step 2802:    A sample is placed in the reaction region 602.
Step 2804:    The meter 604 applies an electrical signal to the working electrode WE.

Step 2806:     The meter 604 measures a first current through the working electrode WE during a first period T1.
Step 2808:     A mediator generates during a second period T2 an intermediate according to the electrical signal.
Step 2810:     The meter 604 measures during a third period T3 a second current through the working electrode WE.
Step 2812:     The meter 604 calculates initial concentration of an analyte according to the first current.
Step 2814:     The meter 604 calculates a diffusion factor of the intermediate in the sample according to the second current.
Step 2816:     The meter 604 corrects the initial concentration of the analyte to generate new concentration of the analyte according to the diffusion factor.
Step 2818:     End.

In Step 2802, the sample includes the analyte (e.g. blood sugar). In Step 2804, the electrical signal applied to the working electrode WE is equal to a voltage drop VWR between the working electrode WE and the reference electrode RE. As shown in FIG. 4, FIG. 10, and FIG. 11, when the sample is placed in the reaction region 602, the mediator in the sample can directly or indirectly seize electrons from the analyte to become a reduced state mediator, wherein concentration of the mediator is much greater than concentration of the analyte. Therefore, in Step 2806, as shown in FIG. 10 and FIG. 12, during the first period T1, the electrical signal applied to the working electrode WE is a positive polarity signal, so the reduced state mediator can transfer electrons to the working electrode WE through a diffusion effect. That is to say, the working electrode WE can generate the first current through the reduced state mediator during the first period T1. The present invention is not limited to the mediator in the sample directly or indirectly seizing electrons from the analyte to become a reduced state mediator. That is to say, the mediator in the sample can also directly or indirectly transfer electrons to the analyte to become an oxidized state mediator.

In Step 2808, as shown in FIG. 10 and FIG. 13, because the concentration of the mediator is much greater than the concentration of the analyte, the majority of mediator not reacted with the analyte can generate reduction reaction on the surface of the working electrode WE when the electrical signal applied to the working electrode WE is a negative polarity signal during the second period T2, resulting in the high concentration reduced state mediator (that is, the intermediate) being accumulated on the surface of the working electrode WE, wherein the concentration of the reduced state mediator accumulated on the surface of the working electrode WE is not influenced by the concentration of the analyte (e.g. blood sugar). In another embodiment of the present invention, because the electrical signal applied to the working electrode WE is a positive polarity signal during the second period T2, the majority of mediator not reacted with the analyte can generate oxidation reaction on the surface of the working electrode WE. That is to say, the high concentration oxidized state mediator can be accumulated on the surface of the working electrode WE. Furthermore, the present invention is not limited to the electrical signal applied to the working electrode WE being a voltage signal during the second period T2, that is, the electrical signal applied to the working electrode WE can also be a current signal during the second period T2.

In Step 2810, diffusion behavior of the mediator in the sample corresponds to the diffusion factor of the sample, wherein the diffusion factor is a function corresponding to a combination of temperature, viscosity, hematocrit, lipemic and ionic strength of the sample. The present invention is not limited to the diffusion factor being a function corresponding to a combination of temperature, viscosity, hematocrit, lipemic and ionic strength of the sample. When the diffusion factor of the mediator in the sample is lower, the reduced state mediator generated during the second period T2 cannot diffuse easily (as shown in FIG. 14); on the other hand, when the diffusion factor of the mediator in the sample is higher, the reduced state mediator generated during the second period T2 can diffuse easily. Therefore, during the third period T3 in FIG. 10, when the electrical signal applied to the working electrode WE is a positive polarity signal, the working electrode WE can generate the greater second current when the diffusion factor of the mediator in the sample is lower (because the more reduced state mediator can be accumulated on the surface of the working electrode WE, the more electrons can be received by the working electrode WE, resulting in the working electrode WE generating the greater second current), and the working electrode WE can generate the smaller second current when the diffusion factor of the mediator in the sample is higher (because the less reduced state mediator can be accumulated on the surface of the working electrode WE, the fewer electrons can be received by the working electrode WE, resulting in the working electrode WE generating the smaller second current). Furthermore, in another embodiment of the present invention, the electrical signal applied to the working electrode WE during the third period T3 is a negative polarity signal opposite to a positive polarity signal during the second period T2.

In Step 2812, the first current during the first period T1 can be used for calculating the initial concentration of the analyte. Furthermore, in Step 2814, because diffusion behavior of the mediator in the sample corresponds to the diffusion factor of the sample, the meter 604 can calculate the diffusion factor of the intermediate (the reduced state mediator) in the sample according to the second current. Finally, in Step 2816, after the diffusion factor is determined, the meter 604 can correct the initial concentration of the analyte to generate the new concentration of the analyte according to the diffusion factor.

Furthermore, during the first period T1, the second period T2, and the third period T3, the counter electrode CE is used for receiving a floating voltage VCE provided by the operational amplifier OP1 to satisfy the first current and the second current generated by the working electrode WE. Therefore, a reaction material can be coated on the surface of the counter electrode CE (or the counter electrode CE can directly react with the electrical signal applied to the working electrode WE) to prevent a voltage of the counter electrode CE from being increased too high during the first period T1, the second period T2, and the third period T3, wherein an oxidized-reduced state of the reaction material coated on the surface of the counter electrode CE is opposite to an original oxidized-reduced state of the mediator in the sample.

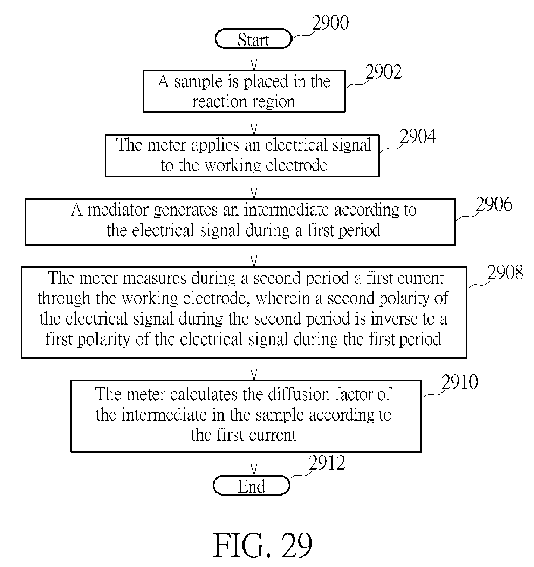

FIG. 29 is a flowchart illustrating a method of utilizing a test strip to detect diffusion factor of a mediator in a sample according to another embodiment. The method in FIG. 29 is illustrated using the test strip 600 in FIG. 4. Detailed steps are as follows:

Step 2900:    Start.
Step 2902:    A sample is placed in the reaction region 602.
Step 2904:    The meter 604 applies an electrical signal to the working electrode WE.
Step 2906:    A mediator generates an intermediate according to the electrical signal during a first period.
Step 2908:    The meter 604 measures during a second period a first current through the working electrode WE, wherein a second polarity of the electrical signal during the second period is inverse to a first polarity of the electrical signal during the first period;
Step 2910:    The meter 604 calculates the diffusion factor of the intermediate in the sample according to the first current.
Step 2912:    End.

A difference between the embodiment in FIG. 29 and the embodiment in FIG. 28 is that in Step 2906, the mediator generates the intermediate during the first period (corresponding to the second period T2 in FIG. 10) according to the electrical signal; in Step 2908, the meter 604 measures the first current (corresponding to the second current in the embodiment in FIG. 28) through the working electrode WE during the second period (corresponding to the third period T3 in FIG. 10); and in Step 2910, the meter 604 calculates the diffusion factor of the intermediate in the sample according to the first current (corresponding to the second current in the embodiment in FIG. 28). Therefore, any configuration which utilizes a second polarity of an electrical signal during a second period inverse to a first polarity of the electrical signal during a first period to detect a diffusion factor of a mediator in a sample falls within the scope of the present invention.

The method of a test strip detecting concentration of an analyte of a sample according to the present invention and the test strip with three electrodes according to the present invention utilize the working electrode to generate a first current for calculating initial concentration of the analyte of the sample according to an electrical signal provided by the meter during a first period, utilize the working electrode to make a mediator in the sample generate reaction according to the electrical signal provided by the meter during a second period, and utilize the working electrode to generate a second current during a third period for calculating a diffusion factor of the mediator in the sample according to the electrical signal provided by the meter. After the diffusion factor of the mediator in the sample is determined, the meter can correct the initial concentration of the analyte in the sample to generate new concentration of the analyte according to the diffusion factor of the mediator in the sample. Therefore, compared to the prior art, the present invention can accurately correct the initial concentration of the analyte in the sample. Furthermore, the method of utilizing a test strip to detect a diffusion factor of a mediator in a sample further provided by the present invention utilizes a second polarity of an electrical signal during a second period inverse to a first polarity of the electrical signal during a first period to detect the diffusion factor of the mediator. Therefore, compared to the prior art, the present invention can rapidly, simply, and accurately detect the diffusion factor of the mediator.

**Claims**

1.   A method of a test strip (100, 600, 1100) detecting concentration of an analyte of a sample, wherein the test strip (100, 600, 1100) comprises a substrate (110, 601) and a reaction region (302, 602), the reaction region (302, 602) comprises a working electrode (WE, 121), a reference electrode (RE, 123), and a counter electrode (CE, 122) and wherein the reaction region (302, 602) is coated with an enzyme , the method **characterized by** the steps of:

placing the sample in the reaction region (302, 602), wherein the analyte reacts with the enzyme to generate a plurality of electrons, and the plurality of electrons are transferred to the working electrode through a mediator;
applying a first direct current (DC) electrical signal to the working electrode during a first period;
measuring a first current through the working electrode during the first period;
applying a second DC electrical signal to the working electrode during a second period;
the mediator generating during the second period an intermediate according to the electrical signal;

applying a third DC electrical signal to the working electrode during a third period;
measuring during the third period a second current through the working electrode, wherein the polarity of the second DC electrical signal during the second period is inverse to the polarity of the first DC electrical signal during the first period and the polarity of the third DC electrical signal during the third period;
calculating an initial concentration of the analyte according to the first current;
calculating a diffusion factor of the intermediate in the sample according to the second current; and
correcting the initial concentration to generate new concentration of the analyte according to the diffusion factor.

2. The method of claim 1, further **characterized in that** when the electrical signal is a voltage, a range of the electrical signal during the first period and a range of the electrical signal during the third period are between 50mV to 1000mV, a range of the electrical signal during the second period is between -50mV to -1000mV, and a range of the second period is between 0.5s to 10s.

3. The method of claim 1 or 2, further **characterized in that** the sample covers at least the working electrode.

4. The method of any one of claims 1-3, further **characterized in that** when the sample is placed in the reaction region (302, 602), the reference electrode receives a reference voltage, and when the electrical signal is a voltage, the electrical signal is equal to a voltage difference between a voltage of the working electrode and the reference electrode.

5. The method of any one of claims 1-4, further **characterized in that** the sample is placed in the reaction region (302, 602), the counter electrode receives a floating voltage to satisfy a current generated by the working electrode during the first period, the second period, and the third period.

6. The method of any one of claims 1-5, further **characterized in that** the intermediate is a reduced state mediator or an oxidized state mediator.

7. The method of any one of claims 1-6, further **characterized in that** the second current is a diffusion current generated by the intermediate during the third period.

8. The method of any one of claims 1-7, further **characterized in that** the second period is after the first period, and the third period is after the second period.

9. The method of any one of claims 1-8, further **characterized in that** the second DC electrical signal is a predetermined current during the second period.

10. The method of any one of claims 1-9, further **characterized in that** the diffusion factor corresponds to a combination of temperature, viscosity, hematocrit, lipemic and ionic strength of the sample.

11. The method of any one of claims 1-10, further **characterized in that** the mediator is coated on the reaction region (302, 602).

12. The method of any one of claims 1-10, further **characterized in that** the mediator is added to the reaction region (302, 602) when the sample is placed in the reaction region (302, 602).

13. The method of claim 1, further **characterized in that** the second DC electrical signal has the polarity and a non-polarity during the second period.

**Patentansprüche**

1. Verfahren eines Teststreifens (100, 600, 1100) zum Nachweis der Konzentration eines Analyten einer Probe, worin der Teststreifen (100, 600, 1100) umfasst, ein Substrat (110, 601) und einen Reaktionsbereich (302, 602), worin der Reaktionsbereich (302, 602) eine Arbeits-Elektrode (WE, 121), eine Referenzelektrode (RE, 123), und eine Gegenelektrode (CE, 122) umfasst, und worin der Reaktionsbereich (302, 602) mit einem Enzym beschichtet ist, wobei das Verfahren durch die Schritte gekennzeichnet ist:

Überführen der Probe in den Reaktionsbereich (302, 602), worin der Analyt mit dem Enzym reagiert, um mehrere Elektronen zu erzeugen, wobei die mehreren Elektronen zu der Arbeits-Elektrode mittels eines Mediators überführt werden;

Anlegen eines ersten elektrischen Gleichstrom- (DC) Signals an die Arbeits-Elektrode während einer ersten Zeitspanne;

Messen eines ersten Stroms durch die Arbeits-Elektrode während der ersten Zeitspanne;

Anlegen eines zweites elektrischen DC-Signals an die Arbeits-Elektrode während einer zweiten Zeitspanne; wobei während der zweiten Zeitspanne ein Zwischenprodukt gemäß dem elektrischen Signal erzeugt;

Anlegen eines dritten elektrischen DC-Signals an die Arbeits-Elektrode während einer dritten Zeitspanne;

Messen eines zweiten Stroms während der dritten Zeitspanne durch die Arbeits-Elektrode, worin eine Polarität des zweiten elektrischen DC-Signals während der zweiten Zeitspanne invers zu dem ersten elektrischen DC-Signal während der ersten Zeitspanne und dem dritten elektrischen DC-Signal während der dritten Zeitspanne ist;

Berechnen einer Anfangs-Konzentration des Analyten gemäß dem ersten Strom;

Berechnen eines Diffusionsfaktors des Zwischenprodukts in der Probe gemäß dem zweiten Strom; und

Korrigieren der Anfangs-Konzentration, um eine neue Konzentration des Analyten gemäß dem Diffusionsfakor zu erzeugen.

2. Verfahren nach Anspruch 1, weiter **dadurch gekennzeichnet, dass** wenn das elektrische Signal eine Spannung ist, ein Bereich des elektrischen Signals während der ersten Zeitspanne und ein Bereich des elektrischen Signals während der dritten Zeitspanne zwischen 50mV bis 1000mV liegt, ein Bereich des elektrischen Signals während der zweiten Zeitspanne zwischen -50mV bis -1000mV, und ein Bereich der zweiten Zeitspanne zwischen 0,5 sek bis 10 sek liegt.

3. Verfahren nach Anspruch 1 oder 2, weiter **dadurch gekennzeichnet, dass** die Probe mindestens die Arbeits-Elektrode bedeckt.

4. Verfahren nach einem der Ansprüche 1-3, weiter **dadurch gekennzeichnet, dass** wenn die Probe in den Reaktionsbereich (302, 602) überführt wird, die Referenzelektrode eine Referenz-Spannung erhält, und wenn das elektrische Signal eine Spannung ist, das elektrische Signal gleich einer Spannungsdifferenz zwischen einer Spannung der Arbeits-Elektrode und der Referenzelektrode ist.

5. Verfahren nach einem der Ansprüche 1-4, weiter **dadurch gekennzeichnet, dass** die Probe in den Reaktionsbereich (302, 602) überführt wird, die Gegenelektrode eine Schwebspannung erhält, um einem Strom zu entsprechen, der durch die Arbeits-Elektrode während der ersten Zeitspanne, der zweiten Zeitspanne, und der dritten Zeitspanne erzeugt wird.

6. Verfahren nach einem der Ansprüche 1-5, weiter **dadurch gekennzeichnet, dass** das Zwischenprodukt ein Mediator in einem reduzierten Zustand oder ein Mediator in einem oxidierten Zustand ist.

7. Verfahren nach einem der Ansprüche 1-6, weiter **dadurch gekennzeichnet, dass** der zweite Strom ein Diffusions-Strom ist, der durch das Zwischenprodukt während der dritten Zeitspanne erzeugt wurde.

8. Verfahren nach einem der Ansprüche 1-7, weiter **dadurch gekennzeichnet, dass** die zweite Zeitspanne nach der ersten Zeitspanne, und die dritte Zeitspanne nach der zweiten Zeitspanne ist.

9. Verfahren nach einem der Ansprüche 1-8, weiter **dadurch gekennzeichnet, dass** das zweite elektrische DC-Signal ein bestimmter Strom während der zweiten Zeitspanne ist.

10. Verfahren nach einem der Ansprüche 1-9, weiter **dadurch gekennzeichnet, dass** der Diffusionsfaktor eine Kombination der Temperatur, Viskosität, Hämatokrit, lipämische und ionische Stärke der Probe ist.

11. Verfahren nach einem der Ansprüche 1-10, weiter **dadurch gekennzeichnet, dass** der Mediator auf den Reaktionsbereich (302, 602) beschichtet ist.

12. Verfahren nach einem der Ansprüche 1-10, weiter **dadurch gekennzeichnet, dass** der Mediator zu dem Reaktionsbereich (302, 602) zugesetzt wird, wenn die Probe in den Reaktionsbereich (302, 602) überführt wird.

13. Verfahren nach Anspruch 1, weiter **dadurch gekennzeichnet, dass** das zweite elektrische DC Signal die Polarität und während der zweiten Zeitspanne eine Nicht-Polarität aufweist.

**Revendications**

1.  Procédé d'une bande de test (100, 600, 1100) détectant une concentration d'un analyte d'un échantillon, dans lequel la bande de test (100, 600, 1100) comprend un substrat (110, 601) et une zone de réaction (302, 602), la zone de réaction (302, 602) comprenant une électrode de travail (WE, 121), une électrode de référence (RE, 123) et une contre-électrode (CE, 122), et dans lequel la zone de réaction (302, 602) est revêtue d'une enzyme, le procédé **se caractérisant par** les étapes suivantes :

    le placement de l'échantillon dans la zone de réaction (302, 602), dans lequel l'analyte réagit avec l'enzyme pour générer une pluralité d'électrons, et la pluralité des électrons étant transférés à l'électrode de travail grâce à un médiateur ;
    l'application d'un premier signal électrique à courant continu (CC) à l'électrode de travail pendant une première période ;
    la mesure d'un premier courant à travers l'électrode de travail pendant la première période ;
    l'application d'un deuxième signal électrique CC à l'électrode de travail pendant une deuxième période ;
    le médiateur générant, pendant la deuxième période, un intermédiaire en fonction du signal électrique ;
    l'application d'un troisième signal électrique CC à l'électrode de travail pendant une troisième période ;
    la mesure, pendant la troisième période, d'un deuxième courant à travers l'électrode de travail,
    dans lequel
    la polarité du deuxième signal électrique CC pendant la deuxième période est inverse à la polarité du premier signal électrique CC pendant la première période et à la polarité du troisième signal électrique CC pendant la troisième période ;
    le calcul d'une concentration initiale de l'analyte en fonction du premier courant ;
    le calcul d'un facteur de diffusion de l'intermédiaire dans l'échantillon en fonction du deuxième courant ; et
    la correction de la concentration initiale pour générer une nouvelle concentration de l'analyte en fonction du facteur de diffusion.

2.  Procédé selon la revendication 1, **caractérisé en outre en ce que**, lorsque le signal électrique est une tension, une plage du signal électrique pendant la première période et une plage du signal électrique pendant la troisième période sont comprises entre 50mV à 1000mV, une plage du signal électrique pendant la deuxième période étant comprise entre -50mV à 1000mV, et une plage de la deuxième période étant comprise entre 0,5s à 10s.

3.  Procédé selon la revendication 1 ou 2, **caractérisé en outre en ce que** l'échantillon recouvre au moins l'électrode de travail.

4.  Procédé selon l'une quelconque des revendications 1-3, **caractérisé en outre en ce que**, lorsque l'échantillon est placé dans la zone de réaction (302, 602), l'électrode de référence reçoit une tension de référence, et lorsque le signal électrique est une tension, le signal électrique étant égal à une différence de tension entre une tension de l'électrode de travail et de l'électrode de référence.

5.  Procédé selon l'une quelconque des revendications 1-4, **caractérisé en outre en ce que** l'échantillon est placé dans la zone de réaction (302, 602), la contre-électrode recevant une tension flottante pour satisfaire à un courant généré par l'électrode de travail pendant la première période, la deuxième période et la troisième période.

6.  Procédé selon l'une quelconque des revendications 1-5, **caractérisé en outre en ce que** l'intermédiaire est un médiateur à l'état réduit ou un médiateur à l'état oxydé.

7.  Procédé selon l'une quelconque des revendications 1-6, **caractérisé en outre en ce que** le deuxième courant est un courant de diffusion généré par l'intermédiaire pendant la troisième période.

8.  Procédé selon l'une quelconque des revendications 1-7, **caractérisé en outre en ce que** la deuxième période est après la première période, et la troisième période étant après la deuxième période.

9.  Procédé selon l'une quelconque des revendications 1-8, **caractérisé en outre en ce que** le deuxième signal électrique CC est un courant prédéterminé pendant la deuxième période.

10. Procédé selon l'une quelconque des revendications 1-9, **caractérisé en outre en ce que** le facteur de diffusion correspond à une combinaison entre une température, une viscosité, une force hématocrite, lipémique et ionique de l'échantillon.

11. Procédé selon l'une quelconque des revendications 1-10, **caractérisé en outre en ce que** le médiateur est revêtu sur la zone de réaction (302, 602).

12. Procédé selon l'une quelconque des revendications 1-10, **caractérisé en outre en ce que** le médiateur est ajouté à la zone de réaction (302, 602) lorsque l'échantillon est placé dans la zone de réaction (302, 602).

13. Procédé selon la revendication 1, **caractérisé en outre en ce que** le deuxième signal électrique CC a la polarité et une non-polarité pendant la deuxième période.

FIG. 1

201

170

150

160

130

120

140

110

FIG. 2

FIG. 3

EP 2 746 759 B1

FIG. 4

FIG. 5

FIG. 6

FIG. 7

FIG. 8

FIG. 9

FIG. 10

0 Mediator

FIG. 11

602

O Mediator

Ø Reduced state
mediator

FIG. 12

602

O Mediator

Ø Reduced state
mediator

FIG. 13

0 Mediator

0 Reduced state
mediator

FIG. 14

Hematocrit

■ 70%
▲ 40%
● 0%

IW
($\mu$A)

Diffusion factor (cm$^2$/s)

FIG. 15

FIG. 16

FIG. 17

FIG. 18

FIG. 19

FIG. 20

FIG. 21

FIG. 22

FIG. 23

FIG. 24

FIG. 25

Bias(%)

100 mg/dL
350 mg/dL

Hematocrit（%）

Before correction

FIG. 26

Bias(%)

100 mg/dL
350 mg/dL

Hematocrit（%）

After correction

FIG. 27

EP 2 746 759 B1

Start — 2800

A sample is placed in the reaction region — 2802

The meter applies an electrical signal to the working electrode — 2804

The meter measures a first current through the working electrode during a first period — 2806

A mediator generates during a second period an intermediate according to the electrical signal — 2808

The meter measures during a third period a second current through the working electrode — 2810

The meter calculates initial concentration of an analyte according to the first current — 2812

The meter calculates a diffusion factor of the intermediate in the sample according to the second current — 2814

The meter corrects the initial concentration of the analyte to generate new concentration of the analyte according to the diffusion factor — 2816

End — 2818

FIG. 28

FIG. 29

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5951836 A
- US 5628890 A
- US 8388821 B
- US 20110139634 A
- WO 2012084194 A1